# EUROPEAN PATENT APPLICATION

(11) **EP 1 792 939 A1**
(43) Date of publication of application: **06.06.2007**
(21) Application number: 06024783.0
(22) Date of filing: 30.11.2006
(51) Int. Cl.: C08L 3/02, C08L 3/08, C08K 5/053, C08K 5/00, A61K 9/48, C08J 5/18

(54) **Blends of gelling and non-gelling starches with gellan gums and plasticizer**

(30) Priority: 02.12.2005 US 293763; 12.05.2006 US 433366
(71) Applicant: National Starch and Chemical Investment Holding Corporation, New Castle, Delaware 19720 (US)
(72) Inventor: Li, Zhixin, Bridgewater New Jersey 08807 (US); Xiao, Chaodong, Boxborough Massachusetts 01719 (US); Huang, Dongyun, Bridgewater New Jersey 08807 (US)
(74) Representative: Held, Stephan

(57) **Abstract**

The present invention relates to a blend of gelling and non-gelling starches with gellan gums and a plasticizer having similar textural and functional properties compared to gelatin. Films prepared using such blends have excellent strength and elongation. The blend can also be used to prepare capsules without heat. The present invention has superior capsule sealability when prepared from such blends or films.

## Description

### FIELD OF THE INVENTION

The invention relates to a gelatin replacement composition. The invention has similar textural and functional properties to gelatin and can be used as a replacement.

### BACKGROUND OF THE INVENTION

The present invention relates to a blend of gelling and non-gelling starches with gellan gums and a plasticizer having similar textural and functional properties compared to gelatin. The present invention also relates to films and soft capsules prepared using such blends and the method of making such films and capsules.

Gelatin is used in various pharmaceutical applications including soft gelatin capsules and hard gelatin capsule shells as well as many different food applications. Soft capsules are used to encapsulate a solution or dispersion, for example of a nutritional or pharmaceutical active agent, in a liquid carrier and have many advantages over other dosage forms, permitting accurate delivery of a unit dose in an easy-to-swallow, transportable, essentially tasteless form.

However, gelatin has many drawbacks, including the cost and continuity of a safe supply. Bovine sources are also undesirable to certain individuals, such as vegetarians and those wishing to maintain Kosher or Halal standards. Further, gelatin is prone to crosslinking, caused by aging or due to reaction with compounds such as aldehydes, which reduces its solubility in gastric juices.

Gelatin provides good sealing of the capsule at a temperature above the melting point of the film, a wet film strong enough to survive the manipulation in the encapsulation machine, dissolution in gastric juices, and sufficient elasticity to allow formation of a capsule. With the growing concern of Bovine Spongiform Encephilitis (BSE) disease in products derived from cows, many attempts have been made to replace gelatin, such as the 25-45% present in soft capsules. However, these approaches have typically failed in that the resultant products had unacceptably different textural and/or functional properties.

Surprisingly, it has now been found that the use of a film forming blend of gelling and non-gelling starches with gellan gums and a plasticizer provides an excellent wet film with high strength and elongation. Also, heat is not required to seal the soft capsules made with these blends. In addition, the soft capsules made with these blends provide superior seal structure.

### SUMMARY OF THE INVENTION

The present invention relates to a blend of gelling and non-gelling starches with different gellan gums and a plasticizer having similar textural and functional properties compared to gelatin and can be used as a replacement thereof. Films prepared using such blends have excellent strength and elongation. The present invention also relates to soft capsules prepared using such blends or films, which do not require heat to be sealed, and have excellent seal structure.

In one embodiment, the blend improved the sealability of the capsules. Sealability is the measurement of seal percentage, seal structure, and burst strength. The capsules made with the present blend gave superior performance in the overall sealability. Seal percentage is defined as the percentage of unbroken capsules after sealing the capsules with filled material. Seal structure is the qualitative examination of the seal with a microscope. Burst strength is the force required to break the capsule.

In another embodiment, heat is not required during the sealing of the capsules made with the present blend. The capsules can be sealed at room temperature without additional heat.

Gelling starch, as used herein, refers to starch with a modulus greater than 500 Pa at 25°C and 10⁻¹ rad/s at 5% solids dissolved in water.

Non-gelling starch, as used herein, refers to starch with a modulus less than 100 Pa at 25°C and 10⁻¹ rad/s at 5% solids dissolved in water.

Gellan gum, as used herein, refers to the extracellular polysaccharide obtained by the aerobic fermentation of the microorganism *Pseudomonas elodea* in a suitable nutrient medium. Various forms of gellan gum have been described in the art and may be used in the present invention.

On a wet basis, as used herein, is intended to include the water in the blend.

Capsule shells, as used here, is intended to mean the capsule without the fill material.

### BRIEF DESCRIPTION OF THE FIGURES

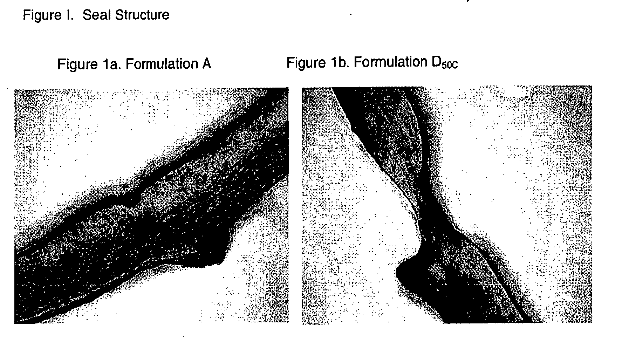

Figure 1 is a micrograph showing the appearance of seal structure of capsule cross sections. Figure 1 a was made from Formulation A; and Figure 1 b was made from Formulation D_{50C}.

### DETAILED DESCRIPTION OF THE INVENTION

The present invention relates to a film-forming blend of gelling and non-gelling starches with different gellan gums and a plasticizer having similar textural and functional properties compared to gelatin. Films prepared using such blends have a high modulus and excellent strength and elongation. The present invention can be used to prepare capsules without heat. The present invention also relates to soft capsule shells prepared using such blends or films, which leads to a superior overall sealability.

The blend of the present invention comprises a gelling starch. Gelling starch is defined as starch with a modulus greater than 500 Pa at 25°C and 10⁻¹ rad/s at 5% solids dissolved in water.

The blend further comprises a non-gelling starch. Non-gelling starch is defined as starch with a modulus less than 100 Pa at 25°C and 10⁻¹ rad/s at 5% solids dissolved in water. In one embodiment, the starches will have a water fluidity in the range of about 65 to 85. Water fluidity is known in the art and, as used herein, is measured using a Thomas Rotational Shear-type Viscometer (commercially available from Arthur A. Thomas Co., Philadelphia, PA), standardized at 30°C with a standard oil having a viscosity of 24.73 cps, which oil requires 23.12±0.05 sec for 100 revolutions.

Starch, as used herein, is intended to include all starches derived from any native source, any of which may be suitable for use herein. A native starch as used herein, is one as it is found in nature. Also suitable are starches derived from a plant obtained by standard breeding techniques including crossbreeding, translocation, inversion, transformation or any other method of gene or chromosome engineering to include variations thereof. In addition, starch derived from a plant grown from artificial mutations and variations of the above generic composition, which may be produced by known standard methods of mutation breeding, are also suitable herein.

Typical sources for the starches are cereals, tubers, roots, legumes and fruits. The native source can be any variety of mung bean, corn (maize), pea, potato, sweet potato, banana, barley, wheat, rice, oat, sago, amaranth, tapioca, arrowroot, canna, sorghum, and waxy and high amylose varieties thereof. As used herein, "waxy" is intended to include a starch containing no more than about 10%, particularly no more than about 5%, more particularly no more than about 3%, and most particularly no more than about 1% amylose by weight. Waxy starches are typically found as waxy maize, waxy pea, waxy wheat, waxy tapioca, waxy rice, waxy barley, waxy potato, and waxy sorghum. As used herein, the term "high amylose" is intended to include a starch containing at least about 40%, particularly at least about 70%, more particularly at least about 80% by weight amylose. As used herein, the term "amylose-containing" is intended to include a starch containing at least about 10% by weight amylose. In one embodiment, suitable starches are those which are amylose containing starches, in another amylose containing starches which are not high amylose.

The starches may be pregelatinized using techniques known in the art and disclosed for example in U.S. Patent Nos. 4,465,702, 5,037,929, 5,131,953, and 5,149,799. Also see, Chapter XXII- "Production and Use of Pregelatinized Starch", Starch: Chemistry and Technology, Vol. III- Industrial Aspects, R.L. Whistler and E.F. Paschall, Editors, Academic Press, New York 1967.

The starch may be a native starch or a modified starch. Modified starch, as used herein, is intended to include starches which have been modified physically, chemically and/or by hydrolysis. Physical modification includes by shearing or thermal-inhibition, for example by the process described in U.S. Patent No. 5,725,676.

The starch may be chemically modified, including without limitation, crosslinked, acetylated, organically esterified, hydroxyethylated, hydroxypropylated, phosphorylated, inorganically esterified, cationic, anionic, nonionic, and zwitterionic, and succinate and substituted succinate derivatives thereof. Such modifications are known in the art, for example in Modified Starches: Properties and Uses, Ed. Wurzburg, CRC Press, Inc., Florida (1986).

The starches may also be converted or hydrolyzed, and suitable starches include fluidity or thin-boiling starches prepared by oxidation, acid hydrolysis, enzyme hydrolysis, heat and or acid dextrinization. These processes are well known in the art.

Any starch having suitable properties for use herein may be purified by any method known in the art to remove starch off flavors and colors that are native to the polysaccharide or created during processing. Suitable purification processes for treating starches are disclosed in the family of patents represented by EP 554 818 (Kasica, et al.). Alkali washing techniques, for starches intended for use in either granular or pregelatinized form, are also useful and described in the family of patents represented by U.S. 4,477,480 (Seidel) and 5,187,272 (Bertalan et al.).

Any native or modified starches that have modulus greater than 500 Pa at 25°C and 10⁻¹ rad/s at 5% solids dissolved in water may be used as a gelling starch in the blend. In one embodiment, suitable gelling starches in the present invention include native mung bean, slightly crosslinked mung bean, slightly crosslinked potato, and slightly crosslinked sago starches. Crosslinked starch is starch that has been modified to form bonds between glucose residues in adjacent starch chains.

Any native or modified starches that have modulus less than 100 Pa at 25°C and 10⁻¹ rad/s at 5% solids dissolved in water may be used as a non-gelling starch in the blend. In one embodiment, suitable non-gelling starches in the present invention include those which are stabilized, including hydroxyalkylated starches such as hydroxypropylated or hydroxyethylated starches, and acetylated starches. In another embodiment, non-gelling starches include dextrinized starches. Yet in another embodiment, starch that has been highly converted is suitable non-gelling starch. In a further embodiment, non-gelling starches include modified waxy and modified high amylose starches. Non-limiting examples of highly converted starches are highly converted sago, highly converted tapioca, and highly converted corn. Converted starch is starch that has been changed to a lower molecular form through various modification. Modifications to convert starch to lower molecular weight are well known in the art.

In one embodiment, the non-gelling starches will have a low viscosity, with a water fluidity in the range of from about 40 to 90. In another embodiment, the starches will have a water fluidity in the range of about 65 to 85. Water fluidity is known in the art and, as used herein, is measured using a Thomas Rotational Shear-type Viscometer (commercially available from Arthur A. Thomas Co., Philadelphia, PA), standardized at 30°C with a standard oil having a viscosity of 24.73 cps, which oil requires 23.12±0.05 sec for 100 revolutions. Accurate and reproducible measurements of water fluidity are obtained by determining the time which elapses for 100 revolutions at different solids levels depending on the starch's degree of conversion: as conversion increases, the viscosity decreases. The conversion may be by any method known in the art including oxidation, enzyme conversion, acid hydrolysis, heat and/or acid dextrinization.

The starches may be used in any amount necessary to achieve the desired viscosity and film thickness. In one embodiment, the total starch will be used in an amount of about 15 to 40%, in another about 20 to 35%, by weight of the composition on a wet basis. In one embodiment, the total starch is added, on a dry weight basis, at a ratio of at least about 6 to 1, and no more than about 60 to 1, by weight of the total gellan. Preferably, the ratio of the gelling to the non-gelling starch is from about 1:25 to about 1:5. More preferably, the ratio of the gelling to the non-gelling starch is from about 1:35 to about 1:6.

The blend of the present invention further comprises at least two gellan gums with different acyl contents, one having a high acyl content and one having a low acyl content. As used herein, high acyl content is intended to mean more than 40% acetyl and more than 45% glyceryl residual substituents per repeat unit. As used herein, low acyl content is intended to mean less than 25% acetyl and less than 15% glyceryl residual substituents per repeat unit.

The high acyl gellan is used to increase the elasticity and is suitably present in an amount of from about 0.3 to 5% by weight of the composition on a wet basis. In another embodiment, the high acyl gellan is present in an amount of about 0.5 to 5% by weight of the composition on a wet basis. The low acyl gellan is used to increase the rigidity and is suitably present in an amount of from about 0.1 to 3% by weight of the composition on a wet basis. In another embodiment, the low acyl gellan is present in an amount of about from about 0.1 to 1 % by weight of the composition on a wet basis. Preferably, the ratio (wt/wt) of high acyl gellan to low acyl gellan is at least about 1 to 1 and no more than about 50 to 1. More preferably, the ratio (wt/wt) of high acyl gellan to low acyl gellan is at least about 4 to 1 and no more than about 30 to 1. The gellan blend may be used in any amount necessary to achieve the desired gel strengthening effect, both modulus and strength and in one embodiment is used in an amount of about 0.4 to 10% by weight of the composition on a wet basis. Alternately, the gellan blend may be used in an amount of about 1 to 5%, by weight of the composition on a wet basis. The gellan is also present to allow heat reversibility of the system, which may be enhanced by decreasing the amount of low acyl gellan to the lower end of the range.

The blend further includes at least one plasticizer. The plasticizer used will depend in part upon the end use application and is intended to include glycerin, sorbitol, sorbitol esters, maltitol, mannitol, xylitol, erythritol, lactitol, propylene glycerol, polyethylene glycol, diethylene glycol, mono acetate of glycerol, diacetate of glycerol, triacetate of glycerol, sucrose, fructose, invert sugars, corn syrup, saccharide oligomers, 1,2-propylenglycol, mono-, di- or triacetates of glycerol, and mixtures thereof. In one suitable embodiment, the plasticizers include glycerin and sorbitol. The plasticizer may be used in any amount necessary to achieve the desired plasticizing effect. Preferably, the ratio (wt/wt) of total starch to plasticizer is at from about 10 to 3 to about 5 to 4. More preferably, the ratio (wt/wt) of total starch to plasticizer is at from about 20 to 7 to about 10 to 7. The plasticizer will be used in an amount of about 10 to 25%, more preferably from about 13 to 22%, by weight of the composition on a wet basis.

In another embodiment, salt buffer is added to the blend. Salt buffers can improve the burst strength of the soft capsules. Further, salt buffers can extend cooking without destabilizing the formulation. Possible salt buffers are, but not limited to, sodium citrate, potassium citrate, sodium phosphate dibasic, and sodium acetate. A range of 0.01 to 1.0% is preferred, by weight of the composition on a wet basis may be used. More preferably, 0.05 to 0.2 %, by weight of the composition on a wet basis, of the salt buffer may be used.

Other additives may optionally be included in the film as is common in the industry as long as they do not adversely affect the film, including without limitation colors, flavors, preservatives, opacifying agents, embrittlement inhibiting agents, and disintegrants. However, the blends are preferably essentially gelatin-free. In one embodiment, the blend contains less than 0.1 % gelatin, in another less than 0.05% gelatin, and in a further embodiment no gelatin.

The blend is advantageous in that it has a hot liquid viscosity suitable for casting on the drum of a rotary die, a process known in the art for producing soft capsule shells. In one embodiment, suitable blends will have a hot viscosity of from about 50,000 to about 300,000 centipoise, in another from about 75,000 to 250, 000 centipoise, at a solids concentration of from about 30 to 70% and a temperature of about 80 to 99°C.

The dry blend is added to water to form a solids concentration suitable for the film or capsule shell process used. For casting of a hot liquid on a cold drum, the concentration is typically suitable at about 30 to 70% solids. Other methods known in the art for forming a film may be used including without limitation extrusion, either direct or from pre-made pellets. The film may be made during the encapsulation process or may be pre-made for later use. The blend can be prepared, and dried to form a film. This can later be formed into soft capsules with heat, water, and/or radiation.

The film's attributes allow it to be used to form essentially gelatin-free capsule shells using techniques known in the art, including on a rotary machine. The soft capsule shells have the same excellent properties as the film and excellent sealability. In some embodiments, the capsule shells can be sealed at moisture contents of about 20 to 60% by weight at room temperature. In further embodiments, the capsule shells can be sealed at moisture contents of about 30 to 55% by weight at room temperature. Excellent seal, as used herein, is intended to mean a seal which will withstand further processing and transportation of the capsule such that it reaches the consumer without leaks or tears.

Capsule shells made using the rotary die process will be similar in look and feel to gelatin capsule shells, having a wet thickness of about 0.25 to 1.8 mm, in another embodiment about 0.5 to 1.4 mm. The fill materials for the soft capsule shells may be any of those typically used in the art, including oils, hydrophobic liquids and emulsions containing active agents. Fill materials may include cosmetics, bath oils, foods, vitamins, detergents, liquids, semisolids, suspensions, flavorings and pharmaceuticals. After filling, the capsules may be dried using techniques conventional in the art, including tray drying.

Unlike the conventional capsules, sealing the capsule shells does not require additional heat. The capsule shells made from the present blend can be sealed at room temperature.

The present blend also leads to overall superior capsule sealability. Sealability has three components: seal percentage, seal structure, and burst strength. The capsules made from the present blend had higher seal percentage. The seal percentage is defined as successful sealing of the capsule shells without leaks, tear or damage at the interface of the seal. The blend also had superior seal structure as observed with a microscope. The blend had higher burst strength, especially with the addition of a buffer salt. The burst strength is defined as the force required to break the capsules.

The following embodiments are presented to further illustrate and explain the present invention and should not be taken as limiting in any regard.

### EXAMPLES

The following examples are presented to further illustrate and explain the present invention and should not be taken as limiting in any regard. All percents used are on a weight basis.

### Example 1 - Modulus of Various Starch Gels

The modulus of four starch gels were measured using five weight percent of starches dissolved in water. Each starch solution was cooked in a 100°C steam bath for 30 minutes. This mixture was cooled overnight, and each gelled starch was cut into a 25mm diameter disk with 2.5mm thickness. The disk was then tested on a TA Instruments ARES RFS, at 25°C, 10⁻¹ rad/s, and 1 % strain. The modulus measurements of various starches are shown in Table 1.

**Table 1. Modulus Measurements of Various Native Starches**

| Starch gel | Mung bean¹ | Corn² | Sago² | Tapioca² |
|---|---|---|---|---|
| Modulus (Pa) | 3,500 | 2,000 | 200 | 38 |

| | | | | |
|---|---|---|---|---|
| ¹Product of SitThiNan, Thailand ²Commercially available from National Starch and Chemical Company, NJ | | | | |

### Example 2 - Formulation of Gelatin-Free Blends

Formulations A-D were made using the following method. Formulation A and B are exemplary formulations; Formulation C borders outside the preferred range of ratios of the gelling and non-gelling starches; Formulation D and E are outside the preferred range. For each sample, the liquid components (plasticizer, buffer salt, water) were mixed and added into a glass bowl of a food mixer (GE, Model #168949). Blended powder ingredients (starches and gellan gums) were added to the bowl, while blending at speed of 1-2. After all of the powders were added, the mixture was further blended for 2 minutes using speed of 3-5 to make a uniform dough. The dough was transferred into a food sealer bag, which was then vacuumed and sealed using a Foodsaver Sealer Professional Ill. The sample was then cooked in a steam bath for 90 to 120 minutes, and kneaded by hand at least every 30 minutes to ensure a uniform melt.

**Table 2 - Components of Gelatin-Free Blends**

| **Blend Material** | **Ingredient** | **A (wt%)** | **B (wt%)** | **C (wt%)** | **D (wt%)** | **E (wt%)** |
|---|---|---|---|---|---|---|
| Gelling starch | Native mung bean starch¹ | 2.00 | 1.50 | 0.50 | 0 | 32.95 |
| Non-gelling starch | Tapioca starch² (PO modified and degraded to 80 water fluidity) | 30.95 | 30.85 | 28.65 | 32.20 | 0 |
| High Acyl Gellan Gum | Kelcogel^{®} LT100³ | 2.00 | 2.50 | 2.50 | 2.75 | 2 |
| Low Acyl Gellan Gum | Kelcogel^{®} F³ | 0.25 | 0.25 | 0.35 | 0.25 | 0.25 |
| Plasticizer | Glycerin⁴ | 19.80 | 19.80 | 18.00 | 19.80 | 19.80 |
| Buffer salt | Sodium citrate buffer salt⁴ | 0 | 0.1 | 0 | 0 | 0 |
| Water | Deionized | 45.00 | 45.00 | 50.00 | 45.00 | 45.00 |

| | | | | | | |
|---|---|---|---|---|---|---|
| ¹SitThiNan, Thailand ²National Starch and Chemical Company, NJ (Water fluidity was measured using a Thomas Rotational Shear-type Viscometer from Arthur A. Thomas Co., PA, standardized at 30°C with a standard oil having a viscosity of 24.73 cps, which oil requires 23.12±0.05 sec for 100 revolutions) ³CP Kelco, IL ⁴Aldrich | | | | | | |

### Example 3 - Preparation of Films and Capsules

Each cooked melt of formulation A-D from Example 2 was poured separately onto a preheated plate (60-105°C). Formulation E could not be poured since the material formed into a solid mass after the cooking process. For very tacky formulations and/or thick films, a thin layer of vegetable oil or other release agent was used on the plate. A preheated (60-105°C) stainless steel applicator, with a 1-2 mm gap, was used to draw a film on the plate.

The capsules were formed from the wet film approximately 2 minutes after casting process.

The wet films were preserved under a moisture sealed condition immediately after the casting and examined for film properties approximately 24 hours later.

### Example 4 - Preparation of Filled Capsules with Vegetable Oil

Each wet film of Example 3 was used to form soft capsules using a bench-top manual capsule press. Vegetable oil was used as an example filling. First, the wet film was placed on the bottom piece of the metal die with a small cavity, and a vacuum was used to conform the film to the cavity surface. The vegetable oil was then added to fill the cavity. Another wet film was placed over the first film and a top piece of the metal die was then used to press against the bottom metal die. A capsule was then formed and removed from the press. The manual press used an air pressure of about 140 psi and a controlled temperature, as shown in Table 3 as the seal temperature.

### Example 5 - Measurement of Capsule Sealability

Each blend was made according to Example 4. Formulations A, B, C, and D_{25C} were sealed at room temperature and 140 psi. Formulation D_{50C} was sealed at 50°C and 140 psi. Formulation E was not tested since this could not be formed into a film.

The seal percent was measured by examining for leaks, tears, or damage of at least thirty capsules filled with vegetable oil. The seal percent indicated the percentage of good seals. As shown in Table 3, A, B, and D₅₀ had 100% seal percentage. The borderline example, C, had 80% seal percentage. Formulation D₂₅ had 0% because this material could not seal at room temperature.

The seal structure was qualitatively determined by examining the cross section of the capsule with the Olympus SZH10 Reflective Microscope. The pictures of the seal structures of Formulation A and D₂₅ capsules are shown in Figure I. Capsules shells were dried for 3 days and a specimen was sliced from cross section of the capsule. Pictures of seal points were taken with a microscope. The width of the specimens was approximately 0.7 mm. Seal point of Figure 1 a showed a stronger seal than that of 1 b. The capsules made with exemplary Formulation A had better structure than capsules made from Formulation D.

Burst strength was measured with Texture Analyzer TZ-XT2. A capsule was placed on the flat platform of the Texture Analyzer, and a probe was discharged at 5 mm/minute to impact the capsule. As shown in Table 3, Formulation B, the material with buffer salt indicated highest burst strength.

**Table 3 - Capsule Sealability**

| Formulation | A | B | C | D*_{25C} | D**_{50C} | E*** |
|---|---|---|---|---|---|---|
| Seal Temperature (°C) | 25 | 25 | 25 | 25 | 50 | N/A |
| Seal Percent (%) | 100 | 100 | 80 | 0 | 100 | N/A |
| Burst Strength (g) | 1650 | 2000 | 950 | N/A | 1700 | N/A |

| | | | | | | |
|---|---|---|---|---|---|---|
| * D₂₅ is formulation D sealed at 25°C ** D₅₀ is formulation D sealed at 50 °C ***Formulation E was not tested since a film could not be formed. | | | | | | |

## Claims

1. A composition comprising:
a. a gelling starch;
b. a non-gelling starch;
c. a high acyl gellan gum;
d. a low acyl gellan gum; and
e. a plasticizer.

2. The composition of claim 1, wherein the gelling starch has a modulus greater than 500 Pa at 25°C and 10⁻¹ rad/s at 5% solids dissolved in water.

3. The composition of claim 2, wherein the gelling starch is selected from the group consisting of native mung bean starch, slightly crosslinked mung bean starch, slightly crosslinked potato starch, slightly crosslinked sago starch, and mixtures thereof.

4. The composition of claim 1, wherein the non-gelling starch has a modulus less than 100 Pa at 25°C and 10⁻¹ rad/s at 5% solids dissolved in water.

5. The composition of claim 4, wherein the non-gelling starch is selected from the group consisting of highly converted and chemically modified sago starch, highly converted and chemically modified tapioca starch, highly converted and chemically modified corn starch, highly converted waxy starch, modified high amylose starch, dextrinized starch, and mixtures thereof.

6. The composition of claim 1, wherein the gelling starch is a native mung bean starch and the non-gelling starch is a highly converted and chemically modified tapioca starch.

7. The composition of claim 1, wherein the ratio of gelling to non-gelling starch is from about 1:35 to about 1:5.

8. The composition of claim 1, wherein the high acyl gellan gum has more than 40% acetyl and more than 45% glyceryl residual substituents per repeat unit.

9. The composition of claim 1, wherein the low acyl gellan gum has less than 25 % acetyl and less than 15% glyceryl residual substituents per repeat unit.

10. The composition of claim 1, wherein the ratio of high acyl gellan to low acyl gellan gum is from about 1:5 to about 50:1.

11. The composition of claim 1, wherein the ratio of gelling and non-gelling starch to plasticizer is from 10:3 to about 5:4.

12. The composition of claim 1, wherein the ratio of total starches to total gellan gums is from about 6:1 to about 60:1

13. The composition of claim 1, wherein the plasticizer is selected from the group consisting of glycerin, sorbitol, sorbitol esters, maltitol, mannitol, xylitol, erythritol, lactitol, propylene glycerol, polyethylene glycol, diethylene glycol, mono acetate of glycerol, diacetate of glycerol, triacetate of glycerol, sucrose, fructose, invert sugars, corn syrup, saccharide oligomers, 1,2- propylenglycol, mono-, di- or triacetates of glycerol, and mixtures thereof.

14. The composition of claim 1, further comprising a salt buffer.

15. The composition of claim 14, wherein the salt buffer is selected from the group consisting of sodium citrate, potassium citrate, sodium phosphate dibasic, sodium acetate, and mixtures thereof.

16. The composition of claim 1, further comprising an optional ingredient selected from the group consisting of colors, flavors, preservatives, opacifying agents, embrittlement inhibiting agents, disintegrants, salt buffers, and mixtures thereof.

17. A gelatin-free film comprising a composition comprising:
a. a gelling starch;
b. a non-gelling starch;
c. a high acyl gellan gum;
d. a low acyl gellan gum; and
e. a plasticizer.

18. The gelatin-free film of claim 17, wherein the ratio of gelling to non-gelling starch is from about 1:35 to about 1:5 and the ratio of high acyl gellan to low acyl gellan gum is from about 1:4 to about 30:1.

19. A gelatin-free capsule shell comprising a composition comprising:
a. a gelling starch;
b. a non-gelling starch;
c. a high acyl gellan gum;
d. a low acyl gellan gum; and
e. a plasticizer.

20. The gelatin-free capsule shell of claim 19, wherein the ratio of gelling to non-gelling starch is from about 1:35 to about 1:5 and the ratio of high acyl gellan to low acyl gellan gum is from about 1:5 to about 50:1.
